Europäisches Patentamt

**European Patent Office**

Office européen des brevets

(11) Publication number: **0 272 923**
**A2**

# (12) EUROPEAN PATENT APPLICATION

(21) Application number: **87311328.6**

(22) Date of filing: **22.12.87**

(51) Int. Cl.⁴: **C 12 N 9/98**
**C 12 N 9/04, A 23 C 9/12,**
**A 23 L 3/00**

(30) Priority: **24.12.86 US 946410**

(43) Date of publication of application:
**29.06.88 Bulletin 88/26**

(84) Designated Contracting States:
**AT BE CH DE ES FR GB GR IT LI LU NL SE**

(71) Applicant: **SUOMEN SOKERI OY**
**Lansituulentie 7**
**SF-02101 Espoo 10 (FI)**

(72) Inventor: **Scott, Don**
**1400 Meacham Road**
**Schaumburg-Illinois (US)**

Saarki, Marja-Leena
Kyllikinportti 2-P O Box 105
00241 Helsinki (FI)

Virtanen, Juoko
Kyllinkinportti 2-P O Box 105
00241 Helsinki (FI)

Szalkucki, Tom
1400 Meacham Road
Schaumburg-Illinois (US)

Farooqui, Kazi
1400 Meacham Road
Schaumburg-Illinois (US)

(74) Representative: **Jones, Ian et al**
POLLAK MERCER & TENCH High Holborn House 52-54
High Holborn
London WC1V 6RY (GB)

(54) **Particulate product including glucose oxidase.**

(57) A dry free flowing particulate product, particularly suitable for addition to food products as an oxygen scavenger, includes glucose, glucose oxidase and optionally, catalase. Also, a dry free flowing particulate product contains a low molecular weight polysaccharide and glucose oxidase in combination with a hydrolytic enzyme or enzymes which are capable of catalyzing the hydrolysis of said polysaccharide into glucose units. Methods of producing such particulate products are also described.

**0 272 923**

**Description**

PARTICULATE PRODUCT INCLUDING GLUCOSE OXIDASE

This invention relates to a particulate product which includes glucose and glucose oxidase, and, optionally, catalase. The invention also relates to a particulate product which contains a low molecular weight polysaccharide and glucose oxidase in combination with a hydrolytic enzyme or enzymes which are capable of catalyzing the hydrolysis of the polysaccharide into glucose units. The invention also relates to methods of producing such particulate products, which are suitable for use as oxygen scavengers for addition to food products.

Many food products are subject to oxidative deterioration in the presence of free oxygen. Oxygen scavenging food ingredients - to prevent such deterioration - are well known in the art. The enzyme glucose oxidase - which catalyzes the oxidation of glucose into glucono delta lactone - has been used to remove or "scavenge" oxygen in certain food contexts. For example, glucose oxidase has been utilized to remove oxygen from soft drinks and other carbohydrate rich foods. (See, Godfrey, T. et al., Industrial Enzymology (McMillan 1982). Glucose oxidase has also been used as an oxygen scavenger in the packaging of meat products (U.S. Patent No. 3,127,274 to Weinke) and packaged, aqueous food products (U.S. Patent No. 3,193,393 to Scott).

The enzyme catalase is sometimes used in combination with glucose oxidase; catalase catalyzes the reaction of hydrogen peroxide into oxygen and water. In the absence of catalase, hydrogen peroxide produced by the oxidation of glucose to glucono delta lactone can build up. In some applications, this build up is desirable because hydrogen peroxide can act as an anti-microbial agent. If excess hydrogen peroxide is not necessary, or desirable, the presence of catalase serves to remove hydrogen peroxide.

The effectiveness of glucose oxidase as an oxygen scavenger, however, depends on the presence of glucose in the treated product or in the oxygen scavenging ingredient itself. The presence of glucose in the ingredient creates stability problems because of the oxidation of glucose to glucono delta lactone catalyzed by glucose oxidase and its subsequent hydrolysis to gluconic acid; the presence of gluconic acid tends to lower the pH, thereby inactivating the enzyme. To prevent this instability, the glucose and glucose oxidase could be separately stored and added; however, the separate addition of two ingredients, such as glucose oxidase and glucose, to a food system is generally inconvenient, impractical and/or uneconomical.

Sequestration of the enzyme (on an inert support) and glucose in an enzyme/glucose blend helps stability, but can decrease the effectiveness of the glucose oxidase, particularly when the treated product is not aqueous; in non-aqueous contexts, the proximity of the glucose particles to the enzyme may be too remote. The prior art discloses the use of glucose/glucose oxidase and glucose/glucose oxidase/catalase systems to remove or scavenge oxygen in food contexts, including systems in which all or part of the enzyme present is absorbed onto an inert support. However, the prior art does not disclose a water soluble, substantially dry enzyme preparation wherein the enzyme present is intimately combined or associated with the glucose particles.

It has now been discovered, however, that a dry, free flowing particulate preparation which is effective as an oxygen scavenger containing glucose, glucose oxidase and optionally containing catalase can be prepared wherein the constituents are intimately combined or associated. This preparation is suitable for use as a food ingredient, and is particularly suitable for use in food products with limited glucose availability. The preparation is stable despite the intimate association of the enzymes and glucose and retains its enzymatic activity over a period of at least three months at room temperature.

It has also been discovered that a dry, free flowing particulate preparation which is effective as an oxygen scavenger containing glucose oxidase, a low molecular weight polysaccharide, and a hydrolytic enzyme or enzymes capable of catalyzing the hydrolysis of said polysaccharide into glucose units can be prepared wherein the consitiuents are intimately combined or associated. An example of an acceptable, low molecular weight polysaccharide is maltodextrin combined preferably with glucoamylase and alpha-amylase. These preparations are particularly stable because the glucose oxidase does not effectively react with the low molecular weight polysaccharides; only when units of glucose are liberated from the polysaccharide by means of hydrolysis catalyzed by the hydrolytic enzyme or enzymes present (e.g. glucoamylase) is the glucose oxidase free to react with glucose. These preparations are also suitable for use as food ingredients, particularly those with limited glucose availability, and exhibit stability during storage without a significant reduction in glucose oxidase or other enzyme activity, and without a significant generation of glucose. These preparations can also contain glucose as well which assists the scavenging ability of the product.

A. Single Enzyme Systems

One aspect of the present invention which is useful as an oxygen scavenger provides for a dry, free flowing particulate product containing glucose, glucose oxidase and optionally containing catalase. The enzymatic activity of the products is high. Also, the product remains stable for a period of at least three months when stored under virtually anaerobic and limited moisture conditions without refrigeration. This product is referred to as a "single enzyme" system because a single enzyme is present to catalyze the reaction which consumes oxygen.

To prepare the product of the present invention containing glucose, an aqueous solution of glucose oxidase and optionally catalase is combined with glucose in a suitable mixing device to form an admixture. The pH of the aqueous solution, or the admixture is buffered to a pH of preferably between about 5.5 and about 7.5, with a pH of about 7 particularly preferred.

The moisture is thereafter removed from the admixture by a method which does not substantially denature the protein present, and does not result in the formation of a substantial amount of gluconic acid. Suitable methods include heating and drying, freeze drying, and solvent precipitation. The free moisture content of a stable final product depends on the method employed. When heating and drying is utilized, a free moisture content of less than about 2 percent by weight based on the weight of the product is acceptable, with a free moisture content of 1 percent being particularly preferred. If freeze drying is utilized, a free moisture content of less than 10 percent by weight, based on the weight of the final product is preferred, with a free moisture content of less than 6 percent being particularly preferred. The moisture content of the product can also be expressed by "water activity". Water activity is determined by a standard method described in "methods of analysis of the Association of Official Analytical Chemists, 13th Ed. (1980) at page 537. When the water activity of the product is less than about 0.4, the product is stable.

The resulting dry products may then be milled, pulverized or otherwise comminuted to reduce particle size depending on the ultimate end use of the product. The dry product is maintained in a controlled environment which is essentially moisture-free and essentially anaerobic to maintain the enzymatic activity of the product.

The resulting products demonstrate a high level of glucose oxidase activity of about 50 to 300 units/gram (U/g) and high levels of other enzymatic activities present, and when properly stored remain stable at room temperature for periods of three months or more. This stability can be increased further by storing at lower temperatures. These products are ideally suited for use as an oxygen scavenger for food products with limited glucose availability such as packaged cheese products and the like.

It is believed that, as a result of this treatment, the enzymes present are intimately associated or combined with glucose particles; some or all or the surface of these particles may be coated with enzyme. Water is removed quickly enough and/or may under such conditions that prevent the significant oxidation of glucose. The resulting glucose/enzyme particles are maintained under conditions which do not permit substantial oxidation of glucose. It is also believed that at least a portion of the glucose oxidase forms an enzyme/substrate complex with the glucose particles, and is available for substantially immediate deoxygenation upon introduction into an oxygen and moisture containing medium.

The final product is particularly adapted for use in non-liquid systems. Because of the nature of the products (i.e. proximity of the enzymes to the glucose and/or the formation of enzyme/substrate complexes), complete solubilization of the product is not required to bring glucose oxidase and glucose in close enough proximity to commence deoxygenation.

B. Double Enzyme Systems

An additional aspect of the present invention which is useful as an oxygen scavenger provides a dry, free flowing particulate product which contains a low molecular weight polysaccharide such as maltodextrin, glucose oxidase, and hydrolytic enzymes such as glucoamylase and alpha-amylase which catalyze the hydrolysis of said polysaccharide into glucose units. The preparation can optionally contain catalase. The enzymatic activity of this preparation is high and is retained for a relatively long period of time when stored under virtually anaerobic and moisture-free conditions.

This particular product is referred to as a double enzyme system because two sequential enzyme catalyzed reactions are required to consume oxygen. The low molecular weight polysaccharide is preferably constituted by glucose or maltose subunits. In this form, the glucose oxidase does not significantly react with the polysaccharide and the preparation has - at this point - limited oxygen scavenging capabilities. The hydrolytic enzyme or enzymes present have the ability to catalyze the cleavage of glucose units, which can in turn react with oxygen (catalyzed by glucose oxidase) to form glucono delta lactone, giving the product an oxygen scavenging capability.

The low molecular weight polysaccharide of choice is maltodextrin consisting of maltose subunits. Glucoamylase is effective as a hydrolytic enzyme in this context because it catalyzed the cleavage of terminal glucose units from maltodextrin. Glucoamylase can also be used in conjunction with another hydrolytic enzyme such as alpha-amylase; this enzyme catalyzes the random hydrolysis of alpha (1-4) linkages of the polysaccharide to yield a mixture of glucose and maltose. A double enzyme system has the potential to be more stable (i.e. less oxidation of free glucose by glucose oxidase will take place under processing and storage conditions) because an initial enzyme catalyzed reaction is required to produce significant levels of free glucose, required - as noted above - to scavenge oxygen.

Catalase is optionally present, for the reasons noted above, in the double enzyme system. In addition, free glucose can be added to enhance the oxygen scavenging capabilities of the product.

To prepare a double enzyme system of the present invention, an aqueous enzyme solution containing glucose oxidase, a hydrolytic enzyme or enzymes and optionally catalase is combined with a low molecular weight polysaccharide in a suitable mixing device to form an admixture. The pH of the aqueous solution or the admixture is buffered to a pH of preferably about 5.5 to about 7.5, with a pH of about 6 to about 7 being particularly preferred.

Moisture is thereafter removed from the admixture by a suitable method which does not substantially denature the protein present, and does not result in the formation of a substantial amount of glucose and/or gluconic acid. Suitable methods include heating and drying, freeze drying and solvent precipitation. The free moisture content of the resulting protein is less than about 10%, with a moisture content of less that 5% being particularly preferred. If moisture content is measured by "water activity", a level of less than about 0.4 is preferred. The resulting dry products may then be milled, pulverized or otherwise comminuted to reduce particle size depending on the end use of the product. The dry comminuted product is maintained in a controlled environment which is essentially moisture free and anaerobic to maintain the enzymatic activities of the product. The product demonstrates a high level of enzymatic activity which makes it ideally suitable for use as an oxygen scavenger, particularly in food products, such as packaged cheese and the like.

As in the case of the single enzyme system, it is believed that the enzymes present are intimately associated or combined with the polysaccharide and glucose present; some or all of the surfaces may be coated with enzyme. However, water is removed quickly enough or under such conditions that prevent the significant hydrolysis of the polysaccharide and/or the oxidation of glucose. The resulting particles are maintained under conditions which do not permit substantial hydrolysis and/or oxidation, resulting in a stable product. It is also believed that enzyme/substrate complexes may be formed which permits immediate hydrolysis and oxidation when the product is introduced into a medium containing oxygen and moisture.

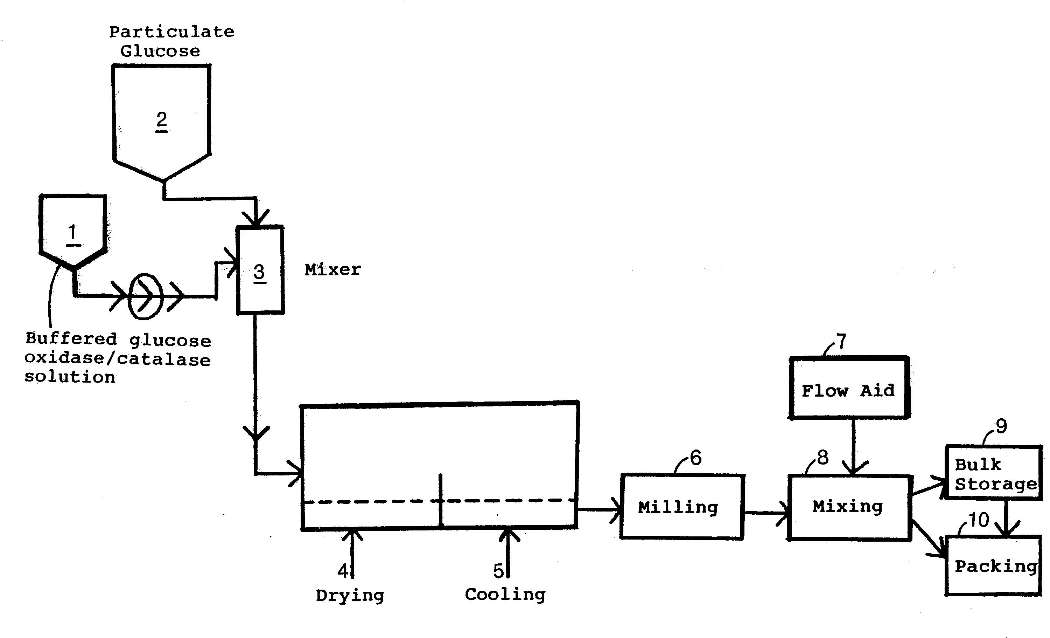

The invention is further described below with reference to the accompanying drawing, the single figure of which is a flow diagram illustrating a method in accordance with the invention for producing an oxygen scavenger food ingredient.

## I. General

The starting materials for the instant invention are an aqueous enzyme solution of containing glucose oxidase, and glucose or a polysaccharide such as maltodextrin. The glucose can be anhydrous or glucose monohydrate, although anhydrous glucose is preferred. Depending on the methods used to mix the protein and glucose, and the methods used to remove water from the resulting admixture, the form of glucose may vary. When a mechanically fluidized bed mixer is used, milled or pulverized glucose powder is preferred. However, glucose in almost any form, e.g., crystalline glucose or high dextrose equivalent corn syrup solids, can be effectively utilized.

The enzymes glucose oxidase, catalase, glucoamylase and alpha-amylase are proteins that are generally available. A typical commercial source for glucose oxidase and catalase is Finnsugar Biochemicals Inc. of Schaumburg, Illinois. The activity of the commercially available glucose oxidase ranges between about 1,000 to about 11,000 units/milliliter (U/ml) and the activity of the catalase ranges between about 40 to about 10,000 U/ml. The activity of commercially available glucoamylase preparations (also available from Finnsugar Biochemicals, Inc.) can vary but is generally about 300 U/ml. The activity of commercially available alpha-amylase preparations is about 85,500 U/ml.

## A. Single Enzyme Systems

In a single enzyme system, glucose and glucose oxidase are present in an admixture.

The pH of the aqueous enzyme solution can be adjusted by addition of a buffer, for example, a citrate or a phosphate citrate buffer to a pH of between about 5.5 to about 7.5, with a pH of about 7 being particularly preferred. NaOH can be used - if necessary - to further adjust the pH. If freeze drying is employed to remove water, the glucose can be present in an aqueous solution buffered to the pH levels set forth above.

If solvent precipitation is utilized to remove water from the enzyme/glucose admixture, the glucose is suspended in the solvent, and a buffered enzyme solution is added thereto.

The glucose and the enzyme solution are combined in a suitable mixer or agitator. In large scale commercial contexts, a mechanically fluidized bed mixer such as the SHUGI mixer can be used. The SHUGI mixer effects almost instantaneous mixing of the constituent elements. Other suitable large scale mixers are available. A fluidized bed drier (such as an AEROMATIC [Model No. SREA 1]) may also be utilized to mix the constituents and to remove the water by drying.

Subsequent to or simultaneously with the mixing of the glucose and the enzyme solution, the water is removed from the resulting glucose/enzyme solution admixture. This must be done in a manner sufficient to prevent a substantial amount of oxidation of glucose to glucono delta lactone and its subsequent hydrolysis to gluconic acid and under conditions which do not substantially denature the protein present.

Oxidation of glucose to glucono delta lactone and its subsequent hydrolysis to gluconic acid will lower the pH of the resulting product in an aqueous environment. If this pH is less than about 5.5, the enzymes present will be partially or wholly inactivated and the deoxygenation capacity of the product will be reduced or eliminated. Hence, water removal must take place quickly, and/or under such conditions which will result in a final product with a pH in an aqueous solution that is not less than about 5.5.

If water removal is carried out under elevated temperatures, the temperature of the admixture or the product must not exceed about 60°C. or the protein present may be substantially denatured. Preferably, the temperature of the admixture or the product should not exceed about 55°C. For example, when a fluidized bed drier is utilized for water removal, it can be operated within various temperature ranges (e.g. 65°C.-45°C) provided the temperature of the admixture or product containing protein does not exceed 60°C., or preferably 55°C.

Water removal can also be carried out by freeze drying. The admixture of glucose and enzyme solution is frozen and the moisture is removed under reduced pressure. Effective and stable oxygen scavenger product

4

was produced with a free moisture content of less than about 10 percent by weight based on the weight of the product, with a free moisture content of about 6 percent by weight being particularly preferred. If moisture content is measured by "water activity", a level of less than about 0.4 is preferred.

Water removal can also be carried out by solvent precipitation using a solvent and conditions which will not substantially inactivate the protein. Suitable solvents include acetone, ethanol and isopropanol. For purposes or precipitating a glucose/enzyme product from an aqueous solution, the preferred ration of solvent to water is 10:1.

Optionally, polyethylene glycol (PEG 4000) can be added either before or during the mixing process; PEG 4000 is used to prevent lumping of the dried product and contributes to the storage stability of the finished product. Optionally, a suitable anti-caking agent can be added during or after the milling, pulverization or comminution of the product, e.g., metal silicates, cellulose powders, microcrystalline cellulose and the like. The choice of a particular agent is determined by the contemplated end use of the product. For use in foods, the preferred agent is microcrystalline cellulose.

Because of the nature of the product it must be maintained under carefully controlled conditions wherein the product's exposure to oxygen and moisture is extremely limited. Maintaining the dry product in a substantially anaerobic and limited moisture environment results in a relatively stable product.

The finished product can be vacuum packed in various containers, e.g., aluminum foil laminate bags, plastic bags or the like. Such bags can also be filled with an inert gas (e.g. nitrogen) to provide a virtually anaerobic environment. At room temperature, properly stored product will remain stable for periods up to three months. Lower storage temperature tends to increase stability.

### B. Double Enzyme Systems

The starting materials for a double enzyme oxygen scavenger include a low molecular weight polysaccharide and an aqueous enzyme solution which contains glucose oxidase, a hydrolytic enzyme or enzymes necessary to catalyze the hydrolysis of the polysaccharide to glucose units and optionally catalase. For purposes of this invention, the preferred polysaccharide is maltodextrin, and the preferred hydrolytic enzymes are glucoamylase and alpha-amylase. The aqueous enzyme solution is buffered to a pH of between about 5.5 and 7.5, with a pH of between about 6 and 7 being particularly preferred.

The enzyme solution and polysaccharide are combined in a suitable mixer or agitator. A SHUGI mixer, or other commercial mixers, effect almost an instantaneous mixing of the constituents. A fluidized bed drier (such as an AEROMATIC [Model No. SREA 1] may also be used to mix the constituents and to remove the water by drying. Spray drying can also be utilized to mix the constituents and dry the product.

Subsequent to, or simultaneously with the mixing of the polysaccharide and enzyme solution, the water is removed from the admixture. This must be done in a manner sufficient to prevent a substantial hydrolysis of the polysaccharide to glucose units and the oxidation of glucose present to glucono-delta-lactone and its subsequent hydrolysis to gluconic acid, and under conditions which do not substantially denature the protein present.

As noted above, the formation of gluconic acid could lower the pH and thereby inactivate the enzymes present. The formation of glucose by hydrolysis of the polysaccharide -which can in turn form gluconic acid in the presence of glucose oxidase and oxygen - should, therefore, be avoided. Moreover, water removal must take place quickly and/or under such conditions so that the pH will be lower than about 5.5, to prevent inactivation of the enzymes present.

As in the case of the single enzyme system, if removal of the water by drying or the like takes place under elevated temperatures, the temperature of the admixture must not exceed about 60°C, and preferably must not exceed about 55°C, to avoid substantial denaturing of the protein present.

Optionally, PEG 4000 can also be added as an anti-caking agent at some point in the process. Other anti-caking or flow agents can be utilized depending on the ultimate end use of the product. The product in its final form must be carefully maintained under conditions which are essentially moisture free and anaerobic; storage under these conditions results in a relatively stable product.

### II. Experimental

EXAMPLE I: Preparation of Oxygen Scavenger (Single Enzyme System) and Evaluation of Stablility

The starting materials in this example were as follows:

1. 475 kg of anhydrous pulverized glucose, with a mean particle size of less than 150 microns and an original moisture content of about 1.3 percent;

2. 17.5 liters of a buffered enzyme solution comprising: 7.3 liters of a glucose oxidase/catalase solution (activities: glucose oxidase 9,450 U/ml, catalase 7,850 U/ml), 0.35 kg PEG 4,000; and 9.9 liters of a 1.5 M citrate/0.95 M phosphate buffer (pH 7)).

The glucose was added to a SHUGI mixer at a rate of about 800 kg/hour. The enzyme solution combined with the buffer was added to the same SHUGI mixer at a rate of 30 liters/hour. The ingredients were mixed by the mixer at room temperature into a substantially homgeneous admixture and dried under gradually decreasing temperatures in the range of 64°C. to 25°C. so that the resulting product had a moisture of about 1 percent. The dry product was then pulverized to a mean particle size of less than 400 microns. The obtained powder was vacuum packed in aluminum foil laminate packages which provided a substantially anaerobic atmosphere. The FIGURE schematically illustrates the method that was used.

The product made by this method showed a high level of glucose oxidase activity, i.e., between about 120 and 130 U/g. The final moisture content of the finished product was approximately 0.8 percent to 1 percent by weight based on the weight of said product. In an aqueous solution the product exhibited a pH of approximately 7.

The oxygen scavenger produced was relatively stable for an extended period of time. Table I sets forth data for oxygen scavenger produced by the method set forth in this experiment stored under different temperatures and packaged under different conditions. This data shows the continued stability of the oxygen scavenger as measured by the activity of the glucose oxidase over a period of seven weeks.

TABLE I

### TABLE I: Stability of Glucose Oxidase/Catalase Glucose Enzyme Preparation

| Storage Conditions | Moisture Content (% by weight) | Glucose Oxidase Activity Measured Over Time (in weeks) | | | | | | | |
|---|---|---|---|---|---|---|---|---|---|
| | | 0 | 1 | 2 | 3 | 4 | 5 | 6 | 7 |
| Room Temperature (RT) | .8 - .9 | 120 | | 106 | 121 | 101 | 115 | | 106 |
| 10°C | .8 - .9 | 130 | | 130 | 130 | 132 | | | 131 |
| vacuum packed in nylon polyethelene packages at room temperature | .8 - .9 | 131 | 133 | 116 | 121 | 130 | 141 | | 124 |
| vacuum packed in aluminum foil laminate package at room temperature | .8 - .9 | 120 | 116 | 116 | 138 | 142 | | | 133 |
| vacuum packed with N₂ fill at room temperature | .8 - .9 | 120 | 122 | 100 | 125 | 145 | 127 | | |

EXAMPLE II: Oxygen Scavenger Composition (Single Enzyme System) with Phosphate/Citrate Buffer

A glucose oxidase, catalase enzyme solution (7.5 ml) was buffered with 7.5 ml of a one molar citrate/phosphate buffer to a final pH of approximately 7. About 15 ml of the enzyme buffer solution was sprayed at a flow rate of 400 ml/minute onto 400 g of pulverized anhydrous glucose at 40°C. in a fluidized bed drier. The product was dried for 2 minutes at 40°C. to produce a dried product with a final moisture content of less than about 1.5 percent. Glucose oxidase activity of this product was approximately 100 to 130 U/g. Dry microcrystalline cellulose was added as an anti-caking agent to constitute approximately 2 percent by weight of the final product, and the product was vacuum packed into an aluminum foil laminate package. The stability of this product was retained for a period of more than three months as measured by the activity of the glucose oxidase.

EXAMPLE III: Oxygen Scavenger Composition (Single Enzyme System) with Citrate Buffer

The starting materials in this example were as follows:

1. 216 kg anhydrous glucose with a mean particle size of less than 150 microns and original moisture content of 1.3 percent; and

2. 7.5 liters of citrate buffered glucose oxidase catalase solution with a pH of 6.1 comprising: 4 liters of an aqueous glucose oxidase (7,00 U/ml) and catalase (3,200 U/ml) solution and 3.5 liters of a citrate buffer (pH 6.6).

The above ingredients were combined in a SHUGI instant mixer. Using feed rates of 800 kg/hour of glucose powder and 30 liters/hour enzyme solution. The mixture was dried in a fluidized bed drier at 55°C. for two hours until the product had a free moisture content of less than about 1 percent by weight based on the dry weight of the product. The product demonstrated high glucose oxidase activity.

The dried product was subsequently milled and vacuum packed in an aluminum foil laminate package where the enzyme product remained stable for a period of three months as measured by the activity of the glucose oxidase.

EXAMPLE IV: Freeze Dried Oxygen Scavenger Composition (Single Enzyme System)

Dextrose monohydrate (105.7 g) and citric acid (anhydrous) (21 g) were dissolved in approximately 600 ml of water. The pH of the resulting solution was adjusted to approximately 6.4 with NaOH. An enzyme solution (150 ml) consisting of glucose oxidase (9,500 U/ml) and catalase (4,000 U/ml) was added, and gently mixed. The resulting admixture was immediately freeze dried in a FTS Systems, Inc. DURA DRY (model No. TDS-3-561-50) freeze drier as follows.

The admixture was poured into trays to a depth of about 20 mm and frozen to about - 50°C. over a period of about 16 hours. The freeze drier chamber was then evacuated to about 60 milli Torr, and the material slowly dried until it reached a final temperature of about 30°C. The condenser temperatures during freeze drying was maintained at about - 80°C. The freeze drying process took about one week.

The freeze dried material was ground with mortar and pestle and the resulting product had a free moisture content of approximately 5 percent by weight based on the weight of the final product, and glucose oxidase activity of about 5.4 U/mg.

EXAMPLE V: Solvent Precipitated Oxygen Scavenger Composition (Single Enzyme System)

Approximately 1,000 g of glucose was suspended in 4,000 ml of acetone at about -20°C. The solution was stirred so that the glucose was evenly suspended. During the stirring process, 150 ml of a solution containing about 1,000 U/ml of glucose oxidase and about 300 U/ml of catalase was added at the rate of about 15 ml/minute. This enzyme solution was buffered with 50 mM sodium phosphate at about a pH of 6.

The acetone containing powder was filtered with the aid of vacuum. The residue was dried until it no longer had a solvent odor. The dry powder was ground with a mortar and pestle. The glucose oxidase activity was about 120 U/g and moisture was less than about 5 percent by weight based on the weight of the final product.

The above examples are intended to be illustrative only and are not intended to limit the scope of this invention. Other variations without departing from the spirit and scope of this invention are possible and will readily present themselves to those skilled in the art.

EXAMPLE VI: Preparation of Oxygen Scavenger (Double Enzyme System) Using a Fluid Bed Drier

A double enzyme system utilizing maltodextrin, glucose oxidase and glucoamylase and/or alpha-amylase was prepared using an AEROMATIC fluid bed drier. In the two examples set forth below in Table II, 200 g of maltodextrin (manufactured by Roquette Freres with a Dextrose Equivalent (DE) of between about 5 to about 8, and a moisture content of about 5%) was Introduced into an AEROMATIC fluid bed drier. The buffered aqueous enzyme solution was sprayed onto the bed of maltodextrin and dried according to the following parameters:

feed rate (enzyme solution)   : 3.5 ml/min
drying time          : 3 min
drying temperature        : 50°-54°C.
outlet temperature         : 24°-28°C.
air flow        : 20 l/min
spraying air        : 7 bar

The activities of the various enzyme solutions added had the following activities:

glucose oxidase/catalase
GO: 11,000 U/ml
Cat: 3,300 U/ml
NaCl: 3M

glucose amylase (SPEZYME GA 300)
GA: 300 U/ml

alpha-amylase (fungal-Novo)
85,500 U/ml

The solutions were buffered to a pH of about 6.1 with 1.3M citrate buffer and .9M phosphate buffer.

## TABLE II: Analysis of Double Enzyme System with Maltodextrin

| | Sample | |
| --- | --- | --- |
| | 1 | 2 |
| **Ingredients Added** | | |
| Maltodextrin, g | 200 | 200 |
| GO/catalase, ml | 3 | 3 |
| Gluco amylase, ml | 4.3 | 4.3 |
| alpha-amylase, ml | - | 3 |
| Buffer (pH = 6.1), ml | 5.0 | 5.0 |
| PEG 6000, g | 0.2 | 0.2 |
| **Analysis of Final Product** | | |
| GO-activity, U/g | 140 | 177 |
| GO-yield (final product), % | 93 | 100 |
| Catalase activity, U/g | 38 | 53 |
| Catalase-yield (final product), % | 80 | 90 |
| Glucose content, % | 0.49 | 0.46 |
| pH | 6.1 | 6.1 |
| Moisture, % | 4.7 | 4.6 |

The data in Table II demonstrates that the technique described above successfully produces a relatively dry, enzyme preparation with a high glucose oxidase activity with minimal or no loss of glucose oxidase activity during preparation. (Catalase was more sensitive to the process and lesser yields were noted in this regard.)

Approximately .005% glucose was present prior to addition of the enzymes; the increase in glucose (to approximatley .5%) is attributable to the action of the hydrolytic enzymes (the glucoamylase and alpha-amylase) during processing. However, the presence of free glucose in these amounts is not considered significant. The addition of alpha-amylase (in Example 2) did not result in higher levels of glucose generated during processing.

Over a period of five weeks, the stability of these samples was analyzed; when stored at low temperatures in a virtually moisture free and anaerobic environment (e.g. in vacuum packed aluminum foil packages), the stability of the samples was observed to be good.

EXAMPLE VII: Preparation of Oxygen Scavenger (Double Enzyme System) Using a Spray Drier

Fifteen liters of a 40% (by weight) maltodextrin solution was fed into a standard commercial spray dryer and a buffered enzyme solution was added just prior to drying. The drier was run with the following parameters:

feed rate of maltodextrin solution:    1.25 l/min
feed rate of buffered enzyme solution:  .19 l/min
drying temperature (out):        69°-74°C.
drying temperature (in):        160°C.

The activities of the various enzyme solutions was added as follows:

glucose oxidase/catalase
GO: 12,500 U/ml
Cat: 4,100 U/ml
NaCl: 3M

glucoamylase (SPEZYME GA 300)
GA activity: 300 U/ml

buffer solution
citrate: .85M
phosphate: .5M
NaCl: .5%
pH: 6.9

Table III set forth below includes data regarding the analysis of the spray dried samples:

### TABLE III:  Analysis of Double Enzyme System with Maltodextrin

| | Sample | |
| | 1 | 2 |
|---|---|---|
| **Ingredients added** | | |
| Maltodextrin, 40% solution | 15 kg | 15 kg |
| GO/catalase | 140 ml | 140 ml |
| Glucoamylase | - | 140 ml |
| Buffer | 2.2 l | 2.2 l |
| PEG 4000 | 8 g | 8 g |
| NaCl | 11 g | 11 g |
| **Analysis of final product** | | |
| Glucose content, % | 0.22 | 0.81 |
| pH | 6.9 | 6.9 |
| Moisture, % | 5.0 | 4.7 |
| GO-yield, % | 63 | 70 |

The increase in the glucose content of the final product is due to the action of the glucoamylase during processing. Spray drying produced samples with acceptable levels of glucose oxidase activity, but the processing was inferior to the fluid bed drier insofar as it related to the stability of glucose oxidase.

Over a period of five weeks, product samples were tested for glucose oxidase activity and the data is set forth below in Table IV. Sample 2, despite the presence of glucoamylase exhibited excellent stability (measured by glucose exodase activity) over time which suggests that the hydrolytic enzymes present are not causing the formation of significant amounts of glucose and/or the glucose oxidase Is unable to catalyze the oxidation of any free glucose.

### TABLE IV:  Stability of Double Enzyme System

| | Sample | |
| | 1 | 2 |
|---|---|---|
| GO-activity U/g | 183 | 202 |
| 1 week | 173 | 200 |
| 2 weeks | 162 | 184 |
| 5 weeks | 170 | 192 |
| Catalase activity U/g | | |
| 2 weeks | | 47 |

EXAMPLE VIII: Formation of Glucose in Maltodextrin Solution

In order to demonstrate the formation of glucose in the presence of maltodextrin and glucoamylase, samples of maltodextrin were incubated 30 minutes with various concentrations of glucoamylase and glucose oxidase. The aqueous maltodextrin solution (45% maltodextrin by weight) was buffered with .16M citrate and .1 potassium phosphate buffer to a pH of about 7. Table V below, sets forth data which shows the increase in

**0 272 923**

the amount of glucose in the presence of glucoamylase. In Sample 8, a buffer with lower capacity (.016M citrate and .01M phosphate) but the same pH exhibited a dramatic increase in glucose production which suggests that the glucoamylase may function better under these conditions.

TABLE V

| Sample | GO-activity added U/g maltodextrin | GA-activity added U/g maltodextrin | Glucose content in solution (mg/100 ml) after incubation | | | Glucose content % based on maltodextrin added |
|---|---|---|---|---|---|---|
| | | | 5 min | 15 min | 30 min | |
| 1. | - | - | 2.7 | 2.6 | 2.8 | 0.005 |
| 2. | 73.5 | - | 3.0 | 4.3 | 5.9 | 0.01 |
| 3. | - | 1.9 | 30 | 52 | 65 | 0.12 |
| 4. | 73.5 | 0.12 | 5.5 | 7.5 | 10.5 | 0.02 |
| 5. | 73.5 | 1.9 | 29 | 48 | 64 | 0.12 |
| 6. | 73.5 | 2.7 | 53 | 59 | 65 | 0.12 |
| 7. | 73.5 | 6.25 | 53 | 61 | 67 | 0.12 |
| 8. | 73.5 | 1.9 | 571 | 1031 | 1114 | 2.1 |

Example IX: Analysis of Oxygen Scavenging Effect of a Double Enzyme System

In order to evaluate the efficacy of a double enzyme oxygen scavenger utilizing maltodextrin, the amount of oxygen removed from cheese samples by products prepared as above was measured. In these tests, cheddar cheese with a moisture content of about 39% (by weight) and pH of about 5.2 was utilized. The cheddar cheese was shredded (fine) and mixed with various enzyme preparations, as detailed below, along with - in some cases - microcrystalline cellulose powder and glucose powder; the cheese and enzyme preparations were mixed thoroughly and placed into an airtight bottle, and stored at about 4°C for 1-3 days. A Servomax 570 $O_2$ analyzer was used to measure headspace oxygen from the sample bottles.

TABLE VI: Oxygen Removal by Double Enzyme System from Cheese Samples

TABLE VI

| Samples | Dosage *% | Glucose powder addition | GO-units/ 0,001 Mol O₂ | Oxygen in headspace 24 hours % | after 2 days % |
|---|---|---|---|---|---|
| **1. Made by fluid bed technique** | | | | | |
| a. GO/MD pH 7.0 | 230 | - | 110 | 10.5 | 8.1 |
| | 50 | 230 | 24 | 0.1 | |
| glucose 0.2% | 2.7 | 150 | 1 | 1.7 | 0.1 |
| b. GO/GA/MD pH 7.0 | 230 | - | 110 | 6.6 | 0.2 |
| | 100 | 50 | 48 | 6.4 | |
| glucose 0.39% | 100 | 150 | 48 | 0.1 | |
| c. GO/GA/MD pH 6.1 | 230 | - | 123 | 0.4 | 0.0 |
| | 100 | 50 | 54 | 0.1 | |
| glucose 0.49% | 50 | 100 | 27 | 0.1 | |
| d. GP/GA/alpha-amylase/ MD pH 6.1 | 230 | - | 150 | 1.4 | 0.0 |
| | 100 | 50 | 65 | 0.1 | |
| glucose 0.46% | 50 | 100 | 33 | 0.1 | |
| **2. Made by spray drying** | | | | | |
| a. GO/GA/MD pH 6.9 glucose 0.22% | 50 | 150 | 34 | 0.1 | |
| b. GO/GA/MD pH 6.9 glucose 0.81% | 230 | - | 176 | 0.1 | |
| | 150 | - | 114 | 2.5 | |
| | 50 | 100 | 38 | 0.1 | |
| | 50 | 150 | 38 | 0.1 | |
| **3. Control GO/dextrose** | 230 | - | 110 | 0.0 | |
| pH 7.0 | 100 | 45 | 53 | 0.0 | |
| | 50 | 100 | 22 | 0.0 | |
| | 2.7 | 150 | 1 | 0.7 | 0.0 |

*% of theoretical demanded glucose content to consume all the oxygen from jar

Table VI sets forth the results of the oxygen scavenging effect of various preparation. Sample 1(b) demonstrates that a double enzyme system utilizing glucose oxidase, glucoamylase and maltodextrin is an effective oxygen scavenger. At lower doses, this product - in combination with glucose powder - was also an effective scavenger. The results also show that a product with a pH of about 6.1 (Sample 1(c)) was more effective than a product with a higher pH of about 7 (Sample 1(b)). The effectiveness of a double enzyme system as an oxygen scavenger is demonstrated by this data, with or without the presence of glucose powder.

**Claims**

1. A method of making a dry free flowing particulate but water-soluble mixture consisting essentially of glucose and glucose oxidase, which comprises the steps of:
combining an aqueous solution of glucose oxidase with glucose to form an admixture;
removing water from said admixture at a rate sufficient to prevent the formation of a substantial amount of gluconic acid so as to produce a dry, particulate substance.

2. A method as claimed in claim 1 wherein catalase is included in the aqueous solution of glucose oxidase which is combined with glucose to form the admixture.

3. A method as claimed in claim 1 or 2 wherein the pH of the admixture is about 7.

4. A method as claimed in claim 1, 2 or 3 wherein the glucose is anhydrous glucose or glucose monohydrate.

5. A method as claimed in claim 1, 2, 3 or 4 wherein the dry, particulate substance has a free moisture content of no more than about 2 percent by weight, based on the weight of the substance.

6. A method as claimed in claim 5 wherein the dry, particulate substance has a free moisture content of no more than about 1 percent by weight, based on the weight of the substance.

7. A method as claimed in any preceding claim wherein an effective amount of an anti-caking agent such as polyethylene glycol, a metal silicate or microcrystalline cellulose is added to the dry, particulate substance.

8. A method as claimed in claim 1, 2, 3 or 4 wherein the water is removed by lyophilization.

9. A method as claimed in claim 8 wherein said dry particulate substance has a free moisture content of less than about 10 percent by weight based on weight of said substance, or a water activity of less than about 0.4.

10. A method of making a dry free flowing particulate but water soluble mixture consisting essentially of a low molecular weight polysaccharide, glucose oxidase and a hydrolytic enzyme or enzymes capable of catalyzing the hydrolysis of the polysaccaride into glucose units, the method comprising the steps of:
combining an aqueous enzyme solution with the polysaccharide to form an admixture;
removing water from the admixture at a rate sufficient to prevent the formation of a substantial amount of glucose and gluconic acid so as to produce a dry, particulate substance.

11. A method as claimed in claim 10 wherein the polysaccharide is maltodextrin, and the hydrolytic enzyme is glucoamylase.

12. A method as claimed in claim 10 or 11 wherein said mixture also includes alpha-amylase.

13. A method as claimed in claim 10, 11 or 12 wherein the pH of the admixture is about 6.

14. A method as claimed in any one of claims 1-12 wherein the pH of the admixture is about 5.5 to about 7.5.

15. A method as claimed in any one of claims 1-7 and 10-14 wherein the water removal is carried out by drying the admixture at a temperature which does not substantially denature the protein that is present.

16. A method as claimed in claim 15 wherein the drying is carried out at a temperature which does not raise the temperature of the admixture or the dry particulate substance to more than about 60° C.

17. A method as claimed in any one of claims 1-4 and 10-13 wherein the dry particulate substance has a free moisture content of no more than about 6 percent by weight based on the weight of the substance.

18. A method as claimed in any preceding claim wherein the dry particulate substance is maintained in an essentially anaerobic environment.

19. A dry free flowing particulate but water soluble mixture consisting essentially of glucose and glucose oxidase and having a free moisture content of less than about 10 percent by weight based on the weight of the mixture, or a water activity of less than about 0.4.

20. A mixture as claimed in claim 19 additionally containing catalase.

21. A mixture as claimed in claim 19 or 20 wherein the free moisture content of the mixture is no more than about 6 percent by weight based on the weight of the mixture.

22. A mixture as claimed in claim 21 wherein the free moisture content of the mixture is no more than about 1 percent by weight based on the weight of the mixture.

23. A dry free flowing particulate but water soluble mixture consisting essentially of a low molecular weight polysaccharide, glucose oxidase, catalase and a hydrolytic enzyme or enzymes capable of catalyzing the hydrolysis of the polysaccharide into glucose units, and having a free moisture content of no more than about 10% by weight based on the weight of the mixture.

24. A mixture as claimed in claim 23 wherein the polysaccharide is maltodextrin.

25. A mixture as claimed in claim 23 or 24 wherein the hydrolytic enzyme is glucoamylase.

26. A mixture as claimed in claim 23, 24 or 25 additionally including alpha-amylase.

27. A mixture as claimed in claim 23, 24, 25 or 26 additionally containing catalase.

28. A mixture as claimed in any one of claims 19-27 including an anti-caking agent.

29. A mixture as claimed in claim 28 wherein the anti-caking agent comprises polyethylene glycol, a metal silicate or microcrystalline cellulose.

30. A hermetically sealed package consisting essentially of a mixture as claimed in any one of claims 19-29 in an essentially anaerobic environment.

31. A package as claimed in claim 30 wherein the anaerobic environment contains gaseous nitrogen.

0272923

Particulate Glucose

Buffered glucose oxidase/catalase solution

2

1

Mixer

3

Drying    Cooling

4    5

Milling

6

Flow Aid

7

Mixing

8

Bulk Storage

9

Packing

10